# EUROPÄISCHE PATENTSCHRIFT

(11) **EP 2 435 082 B1**
(45) Veröffentlichungstag und Bekanntmachung des Hinweises auf die Patenterteilung: **20.05.2020**
(21) Anmeldenummer: 10720724.3
(22) Anmeldetag: 19.05.2010
(51) Int. Cl.: A61K 47/02, A61K 9/70, A61K 31/13

(54) **TRANSDERMALES THERAPEUTISCHES SYSTEM MIT GESTEUERTEM WIRKSTOFFFLUSS, DAS EIN BASISCH REAGIERENDES OXID ENTHÄLT**
TRANSDERMAL THERAPEUTIC SYSTEM WITH A GOVERNED DRUG RELEASE - CONTAINING AN OXIDE OF A BASICAL REACTION
SYSTÈME TRANSDERMIQUE THERAPEUTIQUE À LIBÉRATION GUIDÉE CONTENANT UN OXYDE À RÉACTION BASIQUE

(30) Priorität: 27.05.2009 DE 102009022915
(43) Veröffentlichungstag der Anmeldung: 04.04.2012
(73) Patentinhaber: LTS LOHMANN Therapie-Systeme AG, 56626 Andernach (DE)
(72) Erfinder: EIFLER, René, 56072 Koblenz (DE); KAUFMANN, Regine, 56567 Neuwied (DE); MOHR, Patrick, 53498 Bad Breisig (DE)
(74) Vertreter: Schweitzer, Klaus
(86) Internationale Anmeldenummer: PCT/EP2010/003078
(87) Internationale Veröffentlichungsnummer: WO 2010/136149

(56) Entgegenhaltungen:
- WO-A2-01/43734
- WO-A2-01/43775
- US-A1- 2001 038 862
- US-A1- 2006 093 659
- US-B1- 6 565 879

## Beschreibung

Die vorliegende Erfindung betrifft Zubereitungen zur transdermalen Verabreichung pharmazeutischer Wirkstoffer insbesondere transdermale therapeutische Systeme (TTS). WO0143734, WO0143775, US2006093659, US6565879 legen ein transdermales therapeutisches System offen, dass einen Wirkstoff und ein Oxid enthält. US2001038862 bezieht sich auf ein transdermales System enthaltend ein Peptid und ein Hydroxid freisetzendes Agens. Transdermale therapeutische Systeme sind schichtförmig aufgebaute Darreichungsformen, die mindestens eine wirkstoffhaltige Polymerschicht sowie eine wirkstoffundurchlässige Rückschicht umfassen. Transdermale therapeutische Systeme werden auch als Wirkstoffpflaster bezeichnet. Sie ermöglichen eine lang andauernde Verabreichung des in ihnen enthaltenen pharmazeutischen Wirkstoffs an die Haut oder über die Haut eines zu behandelnden Patienten.

Für eine transdermale Verabreichung von Wirkstoffen über die Haut in therapeutisch erforderlicher Dosis muss der zu verabreichende Wirkstoff eine ausreichende Hautpermeabilität aufweisen. Daher werden bei der Entwicklung von transdermalen therapeutischen Systemen vorzugsweise Wirkstoffe in Form ihrer freien Base (Wirkstoff-Base) verwendet. Wirkstoff-Basen weisen üblicherweise gute, im Vergleich zu den Salzen des entsprechenden Wirkstoffs erheblich bessere Permeationseigenschaften für eine transdermale Verabreichung auf. Zudem lassen sich Wirkstoff-Basen bei der Herstellung von wirkstoffhaltigen Massen für transdermale Zubereitungen gut verarbeiten. Auch aufgrund ihrer schnellen Verfügbarkeit in transdermalen therapeutischen Systemen werden Wirkstoffe in Form ihrer freien Base gegenüber den Salzen des entsprechenden Wirkstoffs bevorzugt verwendet.

Für die resultierenden transdermalen Zubereitungen ergeben sich weitere Vorteile: das Gewicht der Matrix ist geringer und/oder die Wirkstoffkonzentration in der Matrix kann geringer sein, die Konzentration an Permeationsverstärker kann reduziert werden oder es kann völlig auf Permeationsverstärker verzichtet werden, und es lassen sich transdermale therapeutische Systeme mit anderen Klebeeigenschaften herstellen.

Allerdings können die Herstellung transdermaler therapeutischer Systeme mit einem Wirkstoff in Form seiner freien Base sowie transdermale therapeutische Systeme mit einem Wirkstoff in Form seiner freien Base auch Nachteile aufweisen. So muss bereits die Lagerung der Wirkstoff-Base bei niedrigen Temperaturen erfolgen, d. h. bei 4°C oder niedrigeren Temperaturen, um einen unerwünschten Abbau des Wirkstoffs zu vermeiden. Eine Lagerung bei niedrigen Temperaturen ist auch angezeigt, wenn die Wirkstoff-Base leicht flüchtig ist, um zu verhindern, dass der Druck in dem Behälter, in dem die Wirkstoff-Base gelagert wird, zu groß wird.

Probleme können auch bei der Herstellung wirkstoffhaltiger Massen oder beim Beschichten von Trägermaterialien mit diesen wirkstoffhaltigen Massen auftreten. Verwendet man eine Wirkstoff-Base mit einem hohen Dampfdruck (z.B. erkennbar an einer niedrigen Verdunstungszahl), kann es während des Beschichtens und dem nachfolgenden Trocknen der Zubereitung zu erheblichen Wirkstoffverlusten kommen.

Zudem müssen zur Stabilisierung der Wirkstoff-Base häufig Antioxidantien eingesetzt werden, um Wechselwirkungen der Wirkstoff-Base mit anderen Bestandteilen des transdermalen therapeutischen Systems, beispielsweise dem Haftkleber, dem Permeationsverstärker, den Hilfsstoffen, der Rückschicht oder der Schutzfolie, zu verhindern oder zumindest zu verringern.

Unerwünschte Wechselwirkungen einer Wirkstoff-Base sind auch mit der Raumluft möglich. So kann es durch die Luftfeuchtigkeit zu einer Hydrolyse der Wirkstoff-Base kommen. Eine unerwünschte Reaktion der Wirkstoff-Base mit der Luftfeuchtigkeit kann jedoch nur durch ein aufwändiges Herstellungsverfahren vermieden werden, bei dem die transdermalen therapeutischen Systeme ohne Zwischenlagerung und unter Zufuhr eines inerten Gases, beispielsweise Stickstoff oder Helium, anstatt Raumluft in den Packstoffbeutel eingesiegelt werden.

Neben den genannten chemischen Stabilitätsproblemen bei Verwendung einer Wirkstoff-Base können auch physikalische Stabilitätsprobleme auftreten. Häufig ist die Matrix einer Formulierung, bei der eine Wirkstoff-Base anstelle eines entsprechenden Wirkstoff-Salzes verwendet wird, bereits unmittelbar nach der Beschichtung sehr weich. Sofern die Klebkraft nach der Beschichtung sogar noch ansteigen würde, müssen oftmals bereits bei der Herstellung der wirkstoffhaltigen Massen zusätzliche Gerüstbildner zugesetzt werden, damit das transdermale therapeutische System am Ende seiner Applikationsdauer auch wieder vollständig von der Haut entfernt werden kann.

Zudem kann bei transdermalen therapeutischen Systemen mit einer Wirkstoff-Base ein starker sogenannter "kalter Fluss" auftreten, der die Systeme bereits am Inneren ihrer Verpackung anhaften lässt oder die Applikation und das Tragen des Systems erschwert.

Die vorgenannten Probleme bei der Entwicklung und Herstellung von transdermalen therapeutischen Systemen lassen sich zumindest teilweise vermeiden, wenn ein Wirkstoff-Salz anstelle der freien Wirkstoff-Base verwendet wird. Die Verwendung von Wirkstoffen in Form eines ihrer pharmazeutisch annehmbaren Salze für transdermale therapeutische Systeme ist insofern vorteilhaft, als die transdermalen therapeutischen Systeme unempfindlicher auf äußere Einflüsse reagieren, die chemische Stabilität besser ist, weil das Wirkstoff-Salz weniger Wechselwirkungen mit anderen Inhaltsstoffen oder Komponenten des Systems hat und der "kalte Fluss" reduziert ist.

Allerdings ist die Hautpermeabilität von pharmazeutischen Wirkstoffen in Form eines Salzes wesentlich schlechter als die Hautpermeabilität der freien Base dieses Wirkstoffs, so dass viele Wirkstoff-Salze nicht geeignet sind, um Sie mit Hilfe eines transdermalen therapeutischen Systems in therapeutisch erforderlicher Dosis über die Haut verabreichten zu können.

Um jedoch die Fluxrate eines transdermalen therapeutischen Systems auf ein bestimmtes Maß einzustellen, d.h. zu begrenzen, bedarf es bei gut hautpermeablen Wirkstoffen in der Regel einer Steuermembran, die zwischen der Haut und dem Wirkstoffreservoir angeordnet ist. Derartige transdermale therapeutische Systeme erfordern jedoch einen aufwändigen Entwicklungs- und Herstellungsprozess, so dass die Kosten für die Herstellung der Systeme hoch sind. Zudem ändern sich die physikalischen Eigenschaften des Systems wie Flexibilität und Klebkraft durch das Zufügen der Steuermembran.

Die der vorliegenden Erfindung zugrunde liegende Aufgabe bestand daher darin, ein transdermales therapeutisches System bereitzustellen, das die günstigen Permeationseigenschaften von Wirkstoff-Basen nutzt, die Stabilitätsprobleme jedoch vermeidet, wobei deren Fluxrate auf ein vorbestimmtes Maß eingestellt werden kann, ohne dass eine Steuermembran im transdermalen therapeutischen System erforderlich ist.

Die Aufgabe wird durch ein transdermales therapeutisches System laut Anspruch 1 gelöst, bei dem die Matrix oder bei einer mehrschichtigen Matrix mindestens eine der Matrixschichten mindestens einen pharmazeutischen Wirkstoff in Form eines seiner pharmazeutisch annehmbaren Salze und mindestens ein bei Kontakt mit Wasser basisch reagierendes Oxid enthält, so dass es bei Applikation des TTS auf der Haut durch das aus der Haut kommende Wasser zu einer Säure-Base-Reaktion zwischen Wirkstoff-Salz und dem aus dem Oxid entstehenden Hydroxid kommt, bei der der Wirkstoff in Form seiner freien Base als Reaktionsprodukt aus dem TTS freigesetzt werden kann, und gleichzeitig zur Bildung einer schwer wasserlöslichen Schicht des dem Oxid entsprechenden Hydroxids, wodurch der Wirkstofffluss gesteuert wird.

Erst durch das Aufkleben des erfindungsgemäßen TTS auf die Haut kommt es zur Feuchtigkeitsaufnahme in das System und dadurch zu einer Säure-Basen-Reaktion. Für die Reaktionskinetik spielt die Menge an Wasser, die das System aufnimmt/aufgenommen hat, eine Rolle. Prinzipiell muss die Reaktion nur durch Wasser aktiviert werden, da in der Bilanz der Säure-Base-Reaktion das Wasser, das eingesetzt wurde, auch nach der Reaktion wieder zur Verfügung steht. Durch Zufuhr von zusätzlichem Wasser zur Reaktion kann der Ablauf der Reaktion beschleunigt werden.

Der vorliegenden Erfindung liegen die guten Permeationseigenschaften von Wirkstoff-Basen zugrunde. Mit der vorliegenden Erfindung ist es möglich, die Fluxrate für einen Wirkstoff aus einem transdermalen therapeutischen System zu steuern und so einzustellen, dass es in der Regel keiner zusätzlichen Steuermembran bedarf.

Die Fluxrate lässt sich beispielsweise durch Verstärken oder Verringern der Säure-Base-Reaktion steuern. Verändert man in der erfindungsgemäßen Zubereitung die Konzentration des/der Wirkstoff-Salze(s), so ändert sich auch die Kinetik der Säure-Base-Reaktion und somit die Fluxrate. Bei einer höheren Konzentration des Wirkstoff-Salzes ist auch die Fluxrate erhöht. Bei einer niedrigeren Konzentration des/der Wirkstoff-Salze(s) kann auch der Flux verringert werden.

Bei der Konzentration an Oxid verhält sich das System jedoch anders, weil ein zusätzlicher Effekt zu berücksichtigen ist. Wird die Konzentration an Oxid erhöht, beispielsweise über die zur Konzentration des/der Wirkstoff-Salze(s) equimolare Menge hinaus, kann viel Hydroxid gebildet werden, das für eine Reaktion mit dem/den Wirkstoff-Salz(en) zur Verfügung steht. Infolgedessen wird/werden das/die Wirkstoff-Salz(e) in ihre korrespondierenden freien Basen überführt und können über die Haut verabreicht werden. Überschüssiges, schwer in Wasser lösliches Hydroxid bleibt jedoch in der Matrix bzw. Matrixschicht zurück und bildet eine Art Sperrschicht, durch die kein Wasser zu weiterem Oxid vordringen kann, um die weitere Säure-Base-Reaktion zu vermitteln. Dies kann die Wirkstofffreisetzung hemmen. Verringert man die Konzentration so, dass sie kleiner als die equimolare Menge an Wirkstoff-Salz ist, wird die Säure-Base-Reaktion ebenfalls verlangsamt und die Umwandlung des Wirkstoff-Salzes zur Base wird gehemmt.

Bei Ausführungsformen mit einer mehrschichtigen Matrix lässt sich die Fluxrate auch durch eine Koordination der Säure-Base-Reaktion steuern, indem Wirkstoff-Salz(e) und/oder Oxid in verschiedenen Matrixschichten verteilt werden. Arbeitet man das/die Wirkstoff-Salz(e) und das Oxid in verschiedene Matrixschichten ein, so kann der zeitliche Ablauf der Säure-Base-Reaktion gesteuert werden. Hierbei spielt es auch eine Rolle, in welcher Reihenfolge oxidhaltige Matrixschichten und wirkstoffhaltige Matrixschichten aufeinander folgen. Befindet sich beispielsweise die oxidhaltige Matrixschicht vor der wirkstoffhaltigen Matrixschicht, bezogen auf die Richtung, aus der die Feuchtigkeit der Haut in das System eindringt, setzt die Säure-Base-Reaktion schneller ein als wenn die oxidhaltige Matrixschicht erst nach der wirkstoffhaltigen Matrixschicht kommt.

Bei Ausführungsformen mit einer mehrschichtigen Matrix kann die Fluxrate auch durch eine Staffelung der Konzentrationen an Wirkstoff-Salz und/oder Oxid gesteuert werden. Auf diese Weise lässt sich zum Beispiel eine Linearität in der Fluxrate erreichen. Weist die erste Matrixschicht beispielsweise eine niedrige Konzentration an Wirkstoff-Salz und Oxid auf, und die nachfolgenden Schichten eine zunehmende Konzentration an Wirkstoff-Salz und Oxid, lässt sich ein langsames Anfluten des Wirkstoffs und eine gleich bleibende Freisetzung gewährleisten.

Der pharmazeutische Wirkstoff oder die pharmazeutischen Wirkstoffe liegen in der erfindungsgemäßen transdermalen Zubereitung in Form eines oder mehrerer seines/ihrer pharmazeutisch annehmbaren Salzes/Salze vor. Die pharmazeutisch annehmbaren Salze von pharmazeutischen Wirkstoffen sind dem Fachmann bekannt. Als Wirkstoff-Salze kommen insbesondere jene in Betracht, deren Reaktion mit Hydroxiden innerhalb einer Zeitspanne von 0 bis 24 Stunden nach dem Aufbringen der Zubereitung auf die Haut beginnt und deren freie Base eine gute Hautpermeation aufweist.

Besonders geeignet sind die pharmazeutisch annehmbaren Salze von Wirkstoffen
- aus der Gruppe der Antidementia, zum Beispiel Memantinhydrochlorid, Donepezilhydrochlorid, Rivastigmintartrat,
- der Gruppe der Antidepressiva, zum Beispiel Bupropionhydrochlorid, Clomipraminhydrochlorid, Paroxetinhydrochlorid, Sertalinhydrochlorid, Venlafaxinhydrochlorid, Duloxetinhydrochlorid,
- der Gruppe der Wirkstoffe zur Behandlung des Morbus Parkinson und/oder des Restless-Leg-Syndroms, zum Beispiel Amantadinhydrochlorid, Pramipexolhydrochlorid, Ropinirolhydrochlorid, Selegelinhydrochlorid,
- der Gruppe der Narkotika, zum Beispiel Esketaminhydrochlorid, Ketaminhydrochlorid,
- der Gruppe von Wirkstoffen zur Behandlung von ADHS, zum Beispiel Methylphenidathydrochlorid,
- der Gruppe der Schmerzmittel, zum Beispiel Drofeninhydrochlorid, Oxycodonhydrochlorid, Morphinhydrochlorid,
- der Gruppe der Insulin-Sentizizer, zum Beispiel Pioglitazonhydrochlorid,
- der Gruppe der Antihistaminika, zum Beispiel Ceterizinhydrochlorid,
- der Gruppe von Wirkstoffen zur Behandlung der Hypertonie, zum Beispiel Moxonidin,
- der Gruppe von Wirkstoffen zur Behandlung der Hypotonie, zum Beispiel Theodrenalinhydrochlorid,
- der Gruppe von Wirkstoffen zur Behandlung von Harn- und Dranginkontinenz, zum Beispiel Oxybutyninhydrochlorid,
- der Gruppe von Wirkstoffen zur Vorbeugung von Übelkeit und Erbrechen bei Chemotherapien, Strahlentherapien und/oder Operationen, zum Beispiel Palonosetronhydrochlorid, Ondansetronhydrochlorid, Ramosetronhydrochlorid.

Neben den Hydrochloridsalzen kommen auch Sulfate, Phosphate, Silikate, Carbonate, Tartrate, Oxalate und ähnliche Salze der Wirkstoffe in Betracht.

Bei der Verwendung einer Kombination von Wirkstoffen in der erfindungsgemäßen Zubereitung ist das Mengenverhältnis der Wirkstoffsalze untereinander in weiten Bereichen frei wählbar. Ein geeignetes Mengenverhältnis der Wirkstoff-Salze wird vom Fachmann in Abhängigkeit von der bekannten Dosis-Wirkungsbeziehung der jeweiligen Wirkstoffe, im Hinblick auf den gewünschten therapeutischen Effekt sowie die notwendige oder angestrebte Dosierung gewählt. Die jeweils geeignete Tagesdosis eines bekannten Wirkstoffs ist dem Fachmann bekannt oder kann der Fachliteratur entnommen werden.

Der Gehalt an Wirkstoff-Salz(en) in der Matrix beziehungsweise den wirkstoffhaltigen Matrixschichten bei einer mehrschichtigen Matrix sollte zwischen 0,1 und 40 Gew.-% betragen, bezogen auf die Trockenmasse der Matrix beziehungsweise auf die Trockenmasse der wirkstoffhaltigen Matrixschicht.

Die Matrix oder zumindest eine der Matrixschichten der erfindungsgemäßen Zubereitung enthält mindestens ein basisch reagierendes Oxid, das für eine Anwendung auf der Haut geeignet ist. In Betracht kommen die Oxide der Metalle, die mit Wasser zu einer Base oder Lauge reagieren. Hierzu zählen beispielsweise die Oxide der Erdalkalimetalle wie Berylliumoxid, Magnesiumoxid und Calciumoxid.

Der Gehalt an Oxid beträgt vorzugsweise zwischen 0,1 und 40 Gew.-% betragen, bezogen auf die Trockenmasse der Matrix beziehungsweise auf die Trockenmasse der wirkstoffhaltigen Matrixschicht.

In ihrer einfachsten Ausführungsform umfasst die erfindungsgemäße Zubereitung eine einschichtige Matrix, die sowohl mindestens einen pharmazeutischen Wirkstoff in Form eines seiner pharmazeutisch annehmbaren Salze als auch das Oxid enthält.

In bevorzugten Ausführungsformen umfasst die erfindungsgemäße Zubereitung eine mehrschichtige Matrix.

Besonders bevorzugt sind Ausführungsformen mit einer mehrschichtigen Matrix, bei der Wirkstoff und Oxid in verschiedenen Schichten der Matrix enthalten sind.

Dabei kann das erfindungsgemäße System so aufgebaut sein, dass durch unterschiedliche Konzentrationen an Wirkstoff und/oder Oxid in den jeweiligen Schichten ein Wirkstofffluss gezielt eingestellt werden kann. Das Oxid, das bei Kontakt mit Wasser entsprechende Hydroxide bildet, wirkt nicht nur als Steuerelement durch die Umsetzung des Wirkstoff-Salzes in die freie Wirkstoff-Base, sondern auch durch die Bildung einer schwerlöslichen Schicht des entsprechenden Hydroxids, wodurch der Wirkstofffluss ebenfalls reguliert wird.

Die Matrix oder zumindest die Schicht der Matrix, die bei Anwendung des Systems der Haut zugewandt ist, umfasst ein haftklebendes Polymer oder eine Kombination haftklebender Polymere. Unter "haftklebenden Polymeren" werden im Sinne der vorliegenden Beschreibung solche Polymere verstanden, die in Haftkleberformulierungen enthalten sind, und die für eine Anwendung auf der Haut geeignet sind.

Vorzugsweise ist das haftklebende Polymer aus der Gruppe von Polymeren ausgewählt, die aus Polyacrylaten, Polymethacrylaten, Polydimethylsiloxanen, Polyvinylacetaten, Polyisobutylenen, Styrol-Isopren-Styrol-Blockcopolymeren, Styrol-Butadien-Styrol-Blockcopolymeren, Polyterpenen, Ethylen-Vinylacetat-Copolymeren, Kautschuken und Synthesekautschuken besteht.

Der Anteil des/der haftklebenden Polymers/Polymere beträgt vorzugsweise 5 bis 90 Gew.-%, bezogen auf die Matrix beziehungsweise haftklebenden Matrixschichten.

In einer bevorzugten Ausführungsform liegt das haftklebende Polymer oder die haftklebenden Polymere der Matrix in vernetztem Zustand vor. Die Vernetzung der haftklebenden Polymere kann auf dem Fachmann bekannte Weisen erfolgen, beispielsweise durch Verwendung chemischer Vernetzungsmittel, zum Beispiel Aluminium-Acetylacetonat oder TitanAcetylacetonat bei Polyacrylaten, oder mittels Strahlung.

Aufgrund der Zusammensetzung des/der Wirkstoff-Salze und des Oxids kann die erfindungsgemäße Zubereitung zu trocken und nicht in der Lage sein, aus eigenen Kräften an der Haut zu haften. Bei solchen Ausführungsformen kann das System eine zusätzliche Haftkleberschicht aufweisen, beispielsweise auch in Gestalt eines ringförmigen Klebestreifens, der das Wirkstoffreservoir umschließt. Es kann auch von der Maßnahme Gebrauch gemacht werden, dass ein haftklebendes Überpflaster angebracht ist.

Die erfindungsgemäßen TTS weisen eine wirkstoffundurchlassige, okklusive Rückschicht auf. Als Material für die Rückschicht eignen sich insbesondere Polyester, die sich durch eine besondere Festigkeit auszeichnen. Darüber hinaus können auch beliebige andere Kunststoffe für die Herstellung der Rückschicht verwendet werden, beispielsweise Polyvinylchlorid, Ethylenvinylacetat, Vinylacetat, Polyethylen, Polypropylen, Cellulosederivate oder Kombinationen der vorgenannten Polymere. Erforderlichenfalls kann die Rückschicht mit einer zusätzlichen Auflage versehen werden, beispielsweise durch Bedampfen mit einem Metall oder einem anderen diffusionssperrenden Zusatzstoff wie Siliziumdioxid, Aluminiumoxid oder dergleichen.

Für die ablösbare Schutzschicht können dieselben Materialien verwendet werden wie für die Rückschicht, vorausgesetzt, dass sie wieder ablösbar sind. Falls erforderlich kann die Ablösbarkeit einer Folie durch eine geeignete Oberflächenbehandlung erreicht werden, zum Beispiel durch eine Silikonisierung der Folie. Es können aber auch andere ablösbare Schutzschichten verwendet werden, beispielsweise mit Polytetraethylen behandeltes Papier, Cellophan, Polyvinylchlorid oder dergleichen.

Die vorliegende Erfindung erstreckt sich auch auf Verfahren zur Herstellung der erfindungsgemäßen TTS, die sich dadurch auszeichnen, dass mindestens ein pharmazeutisches Wirkstoff-Salz und mindestens ein pharmazeutisch annehmbares, basisch reagierendes Oxid in eine vorzugsweise haftklebende Matrix oder in mindestens eine Matrixschicht eingearbeitet werden. Wirkstoff-Salz und Oxid können in dieselbe polymerhaltige Masse oder in separate, polymerhaltige Massen für die Matrix eingerührt werden. Die resultierenden Massen werden zu Folien verarbeitet, die gegebenenfalls miteinander laminiert werden. Die resultierende Matrix oder das resultierende Laminat wird mit einer Rückschicht und einer wieder ablösbaren Schutzschicht versehen.

Die erfindungsgemäßen TTS erlauben eine Wirkstoffabgabe über einen Zeitraum von mindestens 24 Stunden, vorzugsweise mindestens 72 Stunden, besonders bevorzugt von mindestens 168 Stunden.

Zu Beginn einer Behandlung mit dem erfindungsgemäßen TTS wird ein erstes erfindungsgemäßes TTS auf die Haut des zu behandelnden Patienten appliziert und zur Fortführung der Behandlung wird das applizierte TTS im Abstand von jeweils 24, 72 oder 168 Stunden durch Applizieren eines neuen erfindungsgemäßen TTS ersetzt.

Nachfolgend werden Ausführungsbeispiele aufgeführt:

### Beispiel 1:

| Inhaltsstoffe | Gewicht % |
|---|---|
| Memantin-HCI | 30,0 |
| CaO | 7,5 |
| Acrylpolymer | 62,5 |

### Herstellung:

Die entsprechende Menge an Memantinhydrochlorid wird vorgelegt und in ca. ⅓ der Menge an Wirkstoff mit einem organischen Lösungsmittel (z. B. Ethylacetat) angeschlemmt. Nach anschliessender Zugabe der entsprechenden Menge eines geeigneten Acrylates (z. B. Durotak 9301), National Starch, New Jersey, wird gerührt, um eine homogene Verteilung des Wirkstoffs im Polymer zu gewährleisten.
Calciumoxid wird unter Rühren in die Masse gegeben und weitergerührt, bis eine homogene Suspension entsteht. Die Suspension wird unter starkem Rühren (z. B. Ultra-Turrax) für max. 2 Minuten weiter homogenisiert.
Der Verdunstungsverlust wird gravimetrisch erfasst und mit Ethylacetat ausgeglichen.

Nach Beschichten auf eine geeignete Schutzfolie (z. B. Polyethylenterephthalat-Folie, PET), wird das Laminat getrocknet und eine geeignete okklusive Rückschicht (z. B. PET-Folie) zukaschiert. Nach Stanzen der TTS werden diese in einem geeigneten Material verpackt, bevorzugt PET.

### Beispiel 2:

Das folgende Beispiel zeigt ein Matrixsystem aus mehreren Strichen. Durch den Auftrag mehrerer Striche besteht die Möglichkeit, exakte Wirkstoffprofile einzustellen.
a) 1. Strich

| Inhaltsstoffe | Gewicht % |
|---|---|
| Amantadin-HCI | 29,9 |
| CaO | 7,5 |
| Acrylpolymer | 62,6 |

b) 2. Strich

| Inhaltsstoffe | Gewicht % |
|---|---|
| Amantadin-HCI | 30,0 |
| CaO | 4,6 |
| Acrylpolymer | 65,4 |

### Herstellung:

Die entsprechende Menge an Amantadinhydrochlorid des 1. Striches wird vorgelegt und in ca. ⅓ der Menge an Wirkstoff mit einem organischen Lösungsmittel (z. B. Ethylacetat) angeschlemmt.
Nach anschließender Zugabe der entsprechenden Menge eines geeigneten Acrylates (z. B. GMS 3083, Cytec Industries Inc., New Jersey) wird gerührt, um eine homogene Verteilung des Wirkstoffs im Polymer zu gewährleisten.
Calciumoxid wird unter Rühren in die Masse gegeben und weitergerührt, bis eine homogene Suspension entsteht. Die Suspension wird unter starkem Rühren (z. B. Ultra-Turrax) für max. 2 Minuten weiter homogenisiert.
Der Verdunstungsverlust wird gravimetrisch erfasst und mit Ethylacetat ausgeglichen.

Nach Beschichten des 1. Striches auf eine geeignete Schutzfolie (z. B. PET-Folie), wird das Laminat getrocknet und der 2. Strich zukaschiert. Dieses 2-Schichtlaminat wird ebenfalls getrocknet und anschließend eine geeignete okklusive Rückschicht (z. B. PET-Folie) zukaschiert. Nach Stanzen der TTS werden diese in einem geeigneten Material, bevorzugt PET, verpackt.

## Patentansprüche

1. Transdermales therapeutisches System, umfassend eine wirkstoffundurchlässige, okklusive Rückschicht, eine ein- oder mehrschichtige Matrix und eine abziehbare Schutzfolie, **dadurch gekennzeichnet, dass** die Matrix oder mindestens eine der Matrixschichten einen pharmazeutischen Wirkstoff oder mehrere pharmazeutische Wirkstoffe in Form mindestens eines seiner/ihrer pharmazeutisch annehmbaren Salze und ein pharmazeutisch annehmbares, bei Kontakt mit Wasser basisch reagierendes Oxid enthält, wobei diese Reaktion erst nach der Applikation des TTS auf die Haut durch Aufnahme von Wasser in Gang gesetzt wird und wobei der pharmazeutische Wirkstoff/die pharmazeutischen Wirkstoffe und das Oxid/die Oxide in unterschiedlichen Matrixschichten enthalten sind.

2. Transdermales therapeutisches System nach Anspruch 1, **dadurch gekennzeichnet, dass** oxidhaltige Matrixschichten und wirkstoffhaltige Matrixschichten alternierend aufeinander folgen.

3. Transdermales therapeutisches System nach Anspruch 1 oder 2, **dadurch gekennzeichnet, dass** der Gehalt an Wirkstoff-Salz in der Matrix bzw. der wirkstoffhaltigen Matrixschicht 0,1 bis 40 Gew.-% beträgt, bezogen auf die Trockenmasse der Matrix beziehungsweise auf die Trockenmasse der wirkstoffhaltigen Matrixschicht.

4. Transdermales therapeutisches System nach einem der vorhergehenden Ansprüche, **dadurch gekennzeichnet, dass** das Wirkstoff-Salz aus der Gruppe ausgewählt ist, die aus den pharmazeutisch annehmbaren Hydrachloriden, Sulfaten, Phosphaten, Silikaten, Carbonaten, Tartraten und Oxalaten der Wirkstoffe ausgewählt ist.

5. Transdermales therapeutisches System nach einem der vorangehenden Ansprüche, **dadurch gekennzeichnet, dass** der Gehalt an Oxid in der Matrix beziehungsweise der wirkstoffhaltigen Matrixschichten 0,1 bis 40 Gew.-% beträgt, bezogen auf die Trockenmasse der Matrix beziehungsweise auf die Trockenmasse der wirkstoffhaltigen Matrixschicht.

6. Transdermales therapeutisches System nach einem der vorangehenden Ansprüche, **dadurch gekennzeichnet, dass** das Oxid aus der Gruppe ausgewählt ist, die Berylliumoxid, Magnesiumoxid und Calciumoxid umfasst.

7. Transdermales therapeutisches System nach einem der vorangehenden Ansprüche, **dadurch gekennzeichnet, dass** die Matrix oder mindestens eine der Matrixschichten ein haftklebendes Polymer oder eine Kombination haftklebender Polymere umfasst, wobei bevorzugt das haftklebende Polymer oder die haftklebenden Polymere aus der Gruppe ausgewählt sind, die aus Palyacrylaten, Polymethacrylaten, Polydimethylsiloxanen, Polyvinylacetaten, Polyisobutylenen, Styrol-lsopren-Styrol-Blockcopolymeren, Styrol-Butadien-Styrol-Blockcopolymeren, Polyterpenen, Ethylen-Vinylacetat-Copolymeren, Kautschuken und Synthesekautschuken besteht.

8. Transdermales therapeutisches System nach Anspruch 7, **dadurch gekennzeichnet, dass** das haftklebende Polymer bzw. die haftklebenden Polymere vernetzt sind.

9. Transdermales therapeutisches System nach einem der Ansprüche 7 oder 8, **dadurch gekennzeichnet, dass** der Anteil an haftklebendem Polymer 5 bis 90 Gew.-% beträgt, bezogen auf die Matrix bzw. haftklebende Matrixschicht.

10. Transdermales therapeutisches System nach einem der vorhergehenden Ansprüche, **dadurch gekennzeichnet, dass** es zusätzlich eine haftklebende Schicht aufweist.

11. Transdermales therapeutisches System gemäß Anspruch 2, **dadurch gekennzeichnet, dass** die Matrixschicht oxidhaltig ist, die bei Anwendung des Systems der Haut zugewandt ist.

12. Transdermales therapeutisches System gemäß Anspruch 2, **dadurch gekennzeichnet, dass** die Matrixschicht wirkstoffhaltig ist, die bei Anwendung des Systems der Haut zugewandt ist.

13. Verfahren zur Herstellung eines transdermalen therapeutischen Systems gemäß einem der vorangehenden Ansprüche, **dadurch gekennzeichnet, dass** mindestens ein pharmazeutischer Wirkstoff in Form mindestens eines seiner pharmazeutisch annehmbaren Salze und ein pharmazeutisch annehmbares, basisch reagierendes Oxid in dieselbe polymerhaltige Masse oder in separate, polymerhaltige Massen für die Matrix eingerührt werden, die resultierenden Massen zu Folien, die gegebenenfalls miteinander laminiert werden, verarbeitet werden und die resultierende Matrix oder das resultierende Laminat mit einer Rückschicht und einer wieder ablösbaren Schutzschicht versehen wird.

14. Verwendung eines transdermalen therapeutischen Systems nach einem der Ansprüche 1 bis 12 zu einer transdermalen Wirkstoffabgabe über einen Zeitraum von mindestens 24 Stunden, vorzugsweise mindestens 72 Stunden, besonders bevorzugt von mindestens 168 Stunden.

## Claims

1. Transdermal therapeutic system comprising an active ingredient-impermeable, occlusive backing layer, a single-layer or multilayer matrix, and a removable protective sheet, **characterized in that** the matrix or at least one of the matrix layers contains a pharmaceutical active ingredient or multiple pharmaceutical active ingredients in the form of at least one of its/their pharmaceutically acceptable salts and a pharmaceutically acceptable oxide which reacts to form a base upon contact with water, wherein said reaction is initiated only after the application of the TTS to skin owing to uptake of water and wherein the pharmaceutical active ingredient/the pharmaceutical active ingredients and the oxide/the oxides are contained in different matrix layers.

2. Transdermal therapeutic system according to Claim 1, **characterized in that** oxide-containing matrix layers and active ingredient-containing matrix layers follow one another alternately.

3. Transdermal therapeutic system according to Claim 1 or 2, **characterized in that** the active-ingredient salt content in the matrix or in the active ingredient-containing matrix layer is from 0.1 to 40 % by weight, based on the dry mass of the matrix or on the dry mass of the active ingredient-containing matrix layer.

4. Transdermal therapeutic system according to any of the preceding claims, **characterized in that** the active-ingredient salt is selected from the group which is selected from the pharmaceutically acceptable hydrochlorides, sulfates, phosphates, silicates, carbonates, tartrates and oxalates of the active ingredients.

5. Transdermal therapeutic system according to any of the preceding claims, **characterized in that** the oxide content in the matrix or in the active ingredient-containing matrix layers is from 0.1 to 40 % by weight, based on the dry mass of the matrix or on the dry mass of the active ingredient-containing matrix layer.

6. Transdermal therapeutic system according to any of the preceding claims, **characterized in that** the oxide is selected from the group comprising beryllium oxide, magnesium oxide and calcium oxide.

7. Transdermal therapeutic system according to any of the preceding claims, **characterized in that** the matrix or at least one of the matrix layers comprises a pressure-sensitive adhesive polymer or a combination of pressure-sensitive adhesive polymers, wherein preferably the pressure-sensitive adhesive polymer or the pressure-sensitive adhesive polymers are selected from the group consisting of polyacrylates, polymethacrylates, polydimethylsiloxanes, polyvinyl acetates, polyisobutylenes, styreneisoprene-styrene block copolymers, styrenebutadiene-styrene block copolymers, polyterpenes, ethylene-vinyl acetate copolymers, rubbers and synthetic rubbers.

8. Transdermal therapeutic system according to Claim 7, **characterized in that** the pressure-sensitive adhesive polymer or the pressure-sensitive adhesive polymers are crosslinked.

9. Transdermal therapeutic system according to either of Claims 7 and 8, **characterized in that** the proportion of pressure-sensitive adhesive polymer is from 5 to 90 % by weight, based on the matrix or pressure-sensitive adhesive matrix layer.

10. Transdermal therapeutic system according to any of the preceding claims, **characterized in that** it additionally has a pressure-sensitive adhesive layer.

11. Transdermal therapeutic system according to Claim 2, **characterized in that** the matrix layer facing the skin when using the system contains oxide.

12. Transdermal therapeutic system according to Claim 2, **characterized in that** the matrix layer facing the skin when using the system contains active ingredient.

13. Process for producing a transdermal therapeutic system according to any of the preceding claims, **characterized in that** at least one pharmaceutical active ingredient in the form of at least one of its pharmaceutically acceptable salts and a pharmaceutically acceptable basic oxide with a basic reaction are stirred into the same polymer-containing mass or into separate polymer-containing masses for the matrix, the resulting masses are processed to form sheets which, as appropriate, are laminated to one another, and the resulting matrix or the resulting laminate is provided with a backing layer and a removable protective layer.

14. Use of a transdermal therapeutic system according to any of Claims 1 to 12 for transdermal release of active ingredient over a period of at least 24 hours, preferably at least 72 hours, particularly preferably at least 168 hours.

## Revendications

1. Système thérapeutique transdermique, comprenant une couche de support occlusive imperméable au principe actif, une matrice monocouche ou multicouche et un film protecteur détachable, **caractérisé en ce que** la matrice ou au moins une des couches de matrice contient un principe actif pharmaceutique ou plusieurs principes actifs pharmaceutiques sous la forme d'au moins un de ses/leurs sels pharmaceutiquement acceptable(s) et un oxyde pharmaceutiquement acceptable réagissant comme une base au contact de l'eau, dans lequel ladite réaction n'est initiée par absorption d'eau qu'après l'application du STT sur la peau, et dans lequel le principe actif pharmaceutique ou les principes actifs pharmaceutiques et l'oxyde ou les oxydes sont contenus dans différentes couches de matrice.

2. Système thérapeutique transdermique selon la revendication 1, **caractérisé en ce que** les couches de matrice contenant de l'oxyde et les couches de matrice contenant du principe actif se succèdent de manière alternée.

3. Système thérapeutique transdermique selon la revendication 1 ou 2, **caractérisé en ce que** la teneur en sel de principe actif dans la matrice ou la couche de matrice contenant du principe actif est comprise entre 0,1 et 40 % en poids par rapport à la masse sèche de la matrice ou par rapport à la masse sèche de la couche de matrice contenant du principe actif.

4. Système thérapeutique transdermique selon l'une quelconque des revendications précédentes, **caractérisé en ce que** le sel de principe actif est choisi parmi le groupe constitué des hydrachlorures, sulfates, phosphates, silicates, carbonates, tartrates et oxalates pharmaceutiquement acceptables des principes actifs.

5. Système thérapeutique transdermique selon l'une quelconque des revendications précédentes, **caractérisé en ce que** la teneur en oxyde dans la matrice ou les couches de matrice contenant du principe actif est comprise entre 0,1 et 40 % en poids par rapport à la masse sèche de la matrice ou par rapport à la masse sèche de la couche de matrice contenant du principe actif.

6. Système thérapeutique transdermique selon l'une quelconque des revendications précédentes, **caractérisé en ce que** l'oxyde est choisi parmi le groupe comprenant l'oxyde de béryllium, l'oxyde de magnésium et l'oxyde de calcium.

7. Système thérapeutique transdermique selon l'une quelconque des revendications précédentes, **caractérisé en ce que** la matrice ou au moins une des couches de matrice comprend un polymère adhésif ou une combinaison de polymères adhésifs, dans lequel le polymère adhésif ou les polymères adhésifs est/sont de préférence choisi(s) parmi le groupe consistant en des polyacrylates, polyméthacrylates, polydiméthylsiloxanes, polyacétates de vinyle, polyisobutylènes, copolymères séquencés styrène-isoprène-styrène, copolymères séquencés styrène-butadiène-styrène, polyterpènes, copolymères éthylène-acétate de vinyle, caoutchoucs et caoutchoucs synthétiques.

8. Système thérapeutique transdermique selon la revendication 7, **caractérisé en ce que** le polymère adhésif ou les polymères adhésifs est/sont réticulé(s).

9. Système thérapeutique transdermique selon la revendication 7 ou 8, **caractérisé en ce que** la proportion de polymère adhésif est comprise entre 5 et 90 % en poids par rapport à la matrice ou à la couche de matrice adhésive.

10. Système thérapeutique transdermique selon l'une quelconque des revendications précédentes, **caractérisé en ce qu'**il présente en outre une couche adhésive.

11. Système thérapeutique transdermique selon la revendication 2, **caractérisé en ce que** la couche de matrice tournée vers la peau lors de l'utilisation du système contient de l'oxyde.

12. Système thérapeutique transdermique selon la revendication 2, **caractérisé en ce que** la couche de matrice tournée vers la peau lors de l'utilisation du système contient du principe actif.

13. Procédé de production d'un système thérapeutique transdermique selon l'une quelconque des revendications précédentes, **caractérisé en ce qu'**au moins un principe pharmaceutique actif sous la forme d'au moins un de ses sels pharmaceutiquement acceptables et un oxyde pharmaceutiquement acceptable réagissant comme une base sont mélangés au sein de la même masse contenant du polymère ou au sein de masses contenant du polymère distinctes pour fournir la matrice, les masses résultantes sont transformées en films qui sont éventuellement stratifiés les uns avec les autres, et la matrice résultante ou le stratifié résultant est muni(e) d'une couche de support et d'une couche de protection amovible.

14. Utilisation d'un système thérapeutique transdermique selon l'une quelconque des revendications 1 à 12 pour une administration transdermique de principe actif sur une période d'au moins 24 heures, de préférence d'au moins 72 heures, le plus préférentiellement d'au moins 168 heures.
